# EUROPEAN PATENT APPLICATION

(11) **EP 3 243 824 A1**
(43) Date of publication of application: **15.11.2017**
(21) Application number: 17020198.2
(22) Date of filing: 05.05.2017
(51) Int. Cl.: C07D 487/04, A61K 31/519, A61P 29/00, A61P 35/00

(54) **SOLID FORMS OF IBRUTINIB FREE BASE**

(30) Priority: 11.05.2016 CZ 20160276
(71) Applicant: Zentiva K.S., 102 37 Praha 10 (CZ)
(72) Inventor: Zvonicek, Vit, 170 00 Praha 7 (CZ); Dammer, Ondrej, 253 01 Hostivice (CZ); Krejcik, Lukas, 190 17 Praha 9 (CZ); Schongut, Marek, 140 00 Praha 4 (CZ); Zvatora, Pavel, 198 00 Praha 9 (CZ)
(74) Representative: Jirotkova, Ivana

(57) **Abstract**

The present invention relates to novel solid forms γ, β, α*,* δ, ε and ζ of 1-[(3*R*)-3-[4-amino-3-(4-phenoxyphenyl)-1H-pyrazolo[3,4-d]pyrimidin-1-yl]piperidin-1-yl]prop-2-en-1-one of formula I, known as ibrutinib, and processes for preparing the same. The process for preparing consists in dissolution of ibrutinib free base in a solvent at a temperature from 30°C to the melting point of the respective solvent, crystallization of the solution and isolation of the solid fraction.

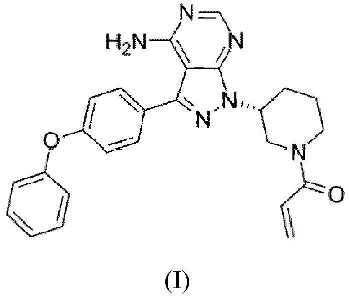

## Description

### Technical Field

The invention relates to novel solid forms of 1-[(3*R*)-3-[4-Amino-3-(4-phenoxyphenyl)-1H-pyrazolo[3,4-d]pyrimidin-1-yl]piperidin-1-yl]prop-2-en-1-one of formula (I), 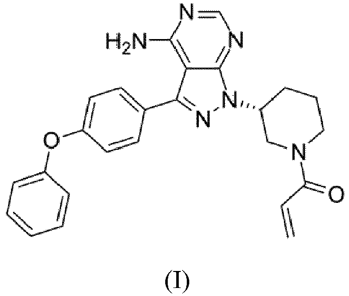 known as ibrutinib, and methods of their preparation.

### Background Art

1-[(3R)-3-[4-Amino-3-(4-phenoxyphenyl)-1H-pyrazolo[3,4-d]pyrimidin-1-yl]piperidin-1-yl]prop-2-en-1-one, which is known as ibrutinib (CAS no. 936563-96-1), belongs to the group of kinase inhibitors that can be used for the treatment of lymphomas. Under the trade name Imbruvica, Ibrutinib was approved by The Food and Drug Administration (FDA) for the treatment of centrocytic lymphoma.

Preparation of this molecule and its isolation with the use of chromatography was described in patent application WO2008/039218. Preparation of crystalline forms of ibrutinib in a solvated or non-solvated form was described in patent documents WO2013/184572, CN 103694241, WO2015/145415 A2 and WO2016/025720 A1. Use of ibrutinib in the treatment of lymphomas together with other pharmaceutically active ingredients was described in patent application WO2013/113841 and patent application WO2013/155347, which also mentions pharmaceutically acceptable salts of this drug without any further description or production example. Besides processes for preparing, patent application WO2013/184572 deals with the pharmaceutical composition of crystalline and solvated forms of ibrutinib for oral administration and mentions pharmaceutically acceptable salts of this drug without any further description or production example. Further use of ibrutinib for the treatment of cancer, inflammatory and autoimmune diseases, together with a description of pharmaceutical compositions mentioning its pharmaceutically acceptable salts without their further description or production example is mentioned in patent application WO2014/004707.

The solubility of the free base of ibrutinib in water is very low, even if aqueous solutions with different pH are used. Discovering new solid phases (polymorphs, solvates and hydrates) of an active pharmaceutical ingredient offers an opportunity to select a suitable modification with more suitable physicochemical properties and processability and improve the characteristics of the chemical product. For this reason, there is an obvious need of novel solid forms (polymorphs, solvates, hydrates) of ibrutinib.

### Disclosure of Invention

The invention discloses solid crystalline forms γ, β, α, δ, ε and ζ of the abrutinib free base, obtainable by recrystallization of the crystalline form A, characterized by the X-ray powder pattern with the use of CuKα radiation having the following reflections: 5.7, 13.6, 16.1 18.9, 21.3, and 21.6±0.1° 2-theta, or of the crystalline form C, characterized by the following reflections: 7.0, 14.0, 15.7, 18.2, 19.1, 19.5, 20.3, 22.1 and 22.9±0.1° 2-theta, from an aliphatic, alicyclic or aromatic solvent.

The crystalline forms A and C are known from the state of art, namely they have been defined in WO2013/184572.

These solid forms of ibrutinib free base of formula I can also be prepared by crystallization, suspension or evaporation of the solvent from a solution of ibrutinib free base in a suitable solvent or mixtures of solvents.

The solvents which might be used for the recrystallization are preferably ketones or aldols; or aliphatic or cyclic ethers; aromatic hydrocarbons or their halo derivatives, especially xylenes, or toluene halo derivatives.

The invention provides crystalline form α of ibrutinib free base, exhibiting the following characteristic reflections in the X-ray powder pattern with the use of CuKα radiation: 6.3; 12.5; 18.0 and 22.9 ± 0.2° 2-theta. In some embodiments, crystalline form α of ibrutinib free base is characterized by the following other reflections in the X-ray powder pattern: 10.3; 17.2; 19.5 and 24.2 ± 0.2° 2-theta. In some embodiments, crystalline form α of ibrutinib free base is further characterized by the differential scanning calorimetry curve with the melting point at 112°C.

The invention further provides a process for preparing crystalline form α of ibrutinib free base, which process comprises:
a) dissolving ibrutinib free base in dioxolane at a temperature in the range from 30°C to the boiling point of the solvent;
b) crystallizing the solution (cooling, evaporation of the solvent), or suspending the solid free base of ibrutinib in the prepared solution;
c) isolating the solid fraction by filtration and its drying.
In some embodiments, the solution is cooled down to -20°C in step b). In some embodiments, solid form C of ibrutinib free base described in patent WO2013/184572 is used in step b). In some embodiments, the suspension is stirred for at least 24 h in step b).

The invention further provides crystalline form β of ibrutinib free base, exhibiting the following characteristic reflections in the X-ray powder pattern with the use of CuKα radiation: 8.0; 10.5; 15.4; 19.0 and 21.1 ± 0.2° 2-theta. In some embodiments, crystalline form β of ibrutinib free base is characterized by the following other reflections in the X-ray powder pattern: 88.9; 17.8; 19.5; 21.7 and 24.5 ± 0.2° 2-theta. In some embodiments, crystalline form β of ibrutinib free base is further characterized by the differential scanning calorimetry curve with the melting point at 88°C.
The invention further provides a process for preparing crystalline form β of ibrutinib free base, which process comprises:
a) dissolving ibrutinib free base in *α,α,α*-trifluorotoluene at a temperature in the range from 30°C to the boiling point of the solvent;
b) crystallizing the solution (cooling, evaporation of the solvent), or suspending the solid free base of ibrutinib in the prepared solution;
c) isolating the solid fraction by filtration and its drying.
In some embodiments, the solution is cooled down to -20°C in step b). In some embodiments, the solid form C of ibrutinib free base described in patent application WO2013/184572 is used in step b). In some embodiments, the suspension is stirred for at least 24 h in step b).

The invention further provides crystalline form γ of ibrutinib free base, exhibiting the following characteristic reflections in the X-ray powder pattern with the use of CuKα radiation: 9.8; 14.1; 17.9; 20.4 and 24.0 ± 0.2° 2-theta. In some embodiments, crystalline form γ of ibrutinib free base is characterized by the following other reflections in the X-ray powder pattern: 9.1; 18.8 and 22.6 ± 0,2° 2-theta. In some embodiments, crystalline form γ of ibrutinib free base is further characterized by the differential scanning calorimetry curve with the melting point at 83°C.

The invention further provides a process for preparing crystalline form γ of ibrutinib free base, which process comprises:
a) dissolving ibrutinib free base in 4-hydroxy-4-methylpentan-2-one at a temperature in the range from 30°C to the boiling point of the solvent;
b) crystallizing the solution (cooling, evaporation of the solvent), or suspending the solid free base of ibrutinib in the prepared solution;
c) isolating of the solid fraction by filtration and its drying.
In some embodiments, the solution is cooled down to -20°C in step b). In some embodiments, the solid form C of ibrutinib free base described in patent application WO2013/184572 is used in step b). In some embodiments, the suspension is stirred for at least 24 h in step b).

The invention further provides crystalline form δ of ibrutinib free base, exhibiting the following characteristic reflections in the X-ray powder pattern with the use of CuKα radiation: 8.5; 16.0; 19.3 and 21.1 ± 0.2° 2-theta. In some embodiments, crystalline form δ of ibrutinib free base is characterized by the following other reflections in the X-ray powder pattern: 7.6; 17.9; 20.0 and 22.0 ± 0.2° 2-theta. In some embodiments, crystalline form δ of ibrutinib free base is further characterized by the differential scanning calorimetry curve with the melting point at 90°C.
The invention further provides a process for preparing crystalline form δ of ibrutinib free base, which process comprises:
a) dissolving ibrutinib free base in *ortho*-xylene at a temperature in the range from 30°C to the boiling point of the solvent;
b) crystallizing the solution (cooling, evaporation of the solvent), or suspending the solid free base of ibrutinib in the prepared solution;
c) isolating the solid fraction by filtration and its drying.
In some embodiments, the solution is cooled down to -20°C in step b). In some embodiments, the solid form C of ibrutinib free base described in patent application WO2013/184572 is used in step b). In some embodiments, the suspension is stirred for at least 24 h in step b).

The invention further provides crystalline form ε of ibrutinib free base, exhibiting the following characteristic reflections in the X-ray powder pattern with the use of CuKα radiation: 6.5; 13.1; 17.5 and 19.9 ± 0.2° 2-theta. In some embodiments, crystalline form ε of ibrutinib free base is characterized by the following other reflections in the X-ray powder pattern: 9.5; 18.3; 20.9 and 22.1 ± 0.2° 2-theta. In some embodiments, crystalline form ε of ibrutinib free base is further characterized by the differential scanning calorimetry curve with the melting point at 95°C.

The invention further provides a process for preparing crystalline form ε of ibrutinib free base, which process comprises:
a) dissolving ibrutinib free base in *meta*-xylene at a temperature in the range from 30°C to the boiling point of the solvent;
b) crystallizing the solution (cooling, evaporation of the solvent), or suspending the solid free base of ibrutinib in the prepared solution;
c) isolating the solid fraction by filtration and its drying.
In some embodiments, the solution is cooled down to -20°C in step b). In some embodiments, the solid form C of ibrutinib free base described in patent application WO2013/184572 is used in step b). In some embodiments, the suspension is stirred for at least 24 h in step b).

The invention further provides crystalline form ζ of ibrutinib free base, exhibiting the following characteristic reflections in the X-ray powder pattern with the use of CuKα radiation: 6.6; 13.1; 17.6 and 19.8 ± 0.2° 2-theta. In some embodiments, crystalline form ζ of ibrutinib free base is characterized by the following other reflections in the X-ray powder pattern: 9.8; 18.4; 20.7; 22.3 and 23.8 ± 0.2° 2-theta. In some embodiments, crystalline form ζ of ibrutinib free base is further characterized by the differential scanning calorimetry curve with the melting point at 99°C.
The invention further provides a process for preparing crystalline form ζ of ibrutinib free base, which process comprises:
a) dissolving ibrutinib free base in *para-xylene* at a temperature in the range from 30°C to the boiling point of the solvent;
b) crystallizing the solution (cooling, evaporation of the solvent), or suspending the solid free base of ibrutinib in the prepared solution;
c) isolating the solid fraction by filtration and its drying.
In some embodiments, the solution is cooled down to -20°C in step b). In some embodiments, the solid form C of ibrutinib free base described in patent application WO2013/184572 is used in step b). In some embodiments, the suspension is stirred for at least 24 h in step b).

The invention further provides the use of crystalline forms α, β, γ, δ, ε and/or ζ of ibrutinib free base for the preparation of a pharmaceutical composition.

The invention further provides a pharmaceutical composition comprising crystalline form α, β, γ, δ, ε and/or ζ of ibrutinib free base and at least one pharmaceutically acceptable excipient.

### Brief Description of Drawings

- Figure 1:: X-ray powder pattern of crystalline form α of ibrutinib free base
- Figure 2:: X-ray powder pattern of crystalline form β of ibrutinib free base
- Figure 3:: X-ray powder pattern of crystalline form γ of ibrutinib free base
- Figure 4:: X-ray powder pattern of crystalline form δ of ibrutinib free base
- Figure 5:: X-ray powder pattern of crystalline form ε of ibrutinib free base
- Figure 6:: X-ray powder pattern of crystalline form ζ of ibrutinib free base

### Detailed description of the invention

Biological availability of active pharmaceutical ingredients greatly depends on whether a crystalline or amorphous product is obtained. An amorphous product is usually more readily soluble; however, it cannot often be obtained in the required quality and it is also often unstable. Conversely, compared to the amorphous form, a crystalline product is often stable, its required purity is easier to achieve and it dissolves more slowly. If solubility of a drug and the related bioavailability needs to be increased, crystalline forms of active pharmaceutical ingredients with a lower melting point, wherein such crystal arrangement guarantees higher solubility of the crystalline form, or amorphous forms of the active pharmaceutical ingredient can advantageously be used. Another possibility of increasing the solubility of a drug is using a crystalline solvate. A solvate refers to such a crystalline form of the active pharmaceutical ingredient that binds a stoichiometric or even non-stoichiometric amount of another chemical substance in its crystal structure, which, in the pure form, occurs in the liquid form at the room temperature. Solvates are generally characterized by increased solubility as compared to non-solvated crystalline forms of active pharmaceutical ingredients.

This invention provides solid forms of ibrutinib free base in a crystalline form, wherein the obtained crystalline forms exhibit a lower melting temperature than the known thermodynamically most stable crystalline form of ibrutinib free base (form A), which was first described in patent application WO2013/184572.

The invention provides pharmaceutically acceptable solid forms of ibrutinib free base and processes for preparing the same. These solid forms of ibrutinib free base can be prepared in corresponding ratios and yields with high chemical purity in a crystalline form. They can be both anhydrous and/or non-solvated, and in the form of hydrates/solvates with the respective solvents.

The prepared solid forms of ibrutinib free base may have various internal arrangements (polymorphism) with different physicochemical properties depending on the conditions of their preparation.

Preparation of the solid forms of ibrutinib free base of formula I according to the present invention is done by crystallization, precipitation or evaporation from a solution of ibrutinib free base in a suitable solvent or mixtures of solvents. Ketones, esters, ethers, amides, nitriles or organic acids, alcohols, aliphatic and aromatic hydrocarbons, chlorinated hydrocarbons, water or their mixtures can be used as suitable solvents. Aliphatic C₁-C₄ alcohols, substituted aromatic solvents, ketones or their mixtures are preferred. The other preferred solvents are ketones or aldols; or aliphatic or cyclic ethers; aromatic hydrocarbons or their halo derivatives, especially xylenes, or toluene halo derivatives. The most commonly used solvents are dioxolane, 4-hydroxy-4-methylpentan-2-one, *α,α,α*-trifluorotoluene, *ortho*-xylene, *meta-*xylene, *para*-xylene or their mixtures.

The final product is typically precipitated or crystallized at temperatures in the range from -20°C to the boiling point of the solvent.

Out of the described solid forms it is form γ of ibrutinib free base that is preferred. During the preparation of crystalline form γ of ibrutinib free base, 4-hydroxy-4-methylpentan-2-one is used as the solvent, which belongs to the group of environment-friendly solvents, which represents an advantage when this molecule is prepared in the industrial scale. At the same time, form γ of ibrutinib free base exhibits approximately 8 times higher dissolution rate as compared to the most stable form of ibrutinib free base (form A) described in patent application WO2013/184572. The dissolution rate of form A in an aqueous solution having pH 2, measured using the method of true dissolution from a disk, is 0.017 mg·min⁻¹·cm⁻² and the dissolution rate of form γ measured under identical conditions is 0.144 mg·min⁻¹·cm⁻².

Also, form β of ibrutinib free base exhibits a higher dissolution rate, compared to form A of ibrutinib free base, namely 0.059 mg·min⁻¹·cm⁻².

The higher dissolution rate makes it possible to formulate treatment drugs with a lower content of the active substance while maintaining the same efficiency and bioavailability of the drug, which brings patients higher usage comfort, a lower risk of undesired effects related to a high dose of the particular drug and at the same time reduces release of unmetabolized drugs into the environment.

Preparation and isolation of ibrutinib free base was first described in patent application WO2008/039218. Patent applications WO2013/184572, CN 103694241, WO2015/145415 A2 and WO2016/025720 A1 have described methods of preparation and characterization of solvated as well as non-solvated forms of ibrutinib free base, as well as its amorphous forms.

Ibrutinib free base (form C) was prepared according to the process disclosed in patent application WO2013/184572.

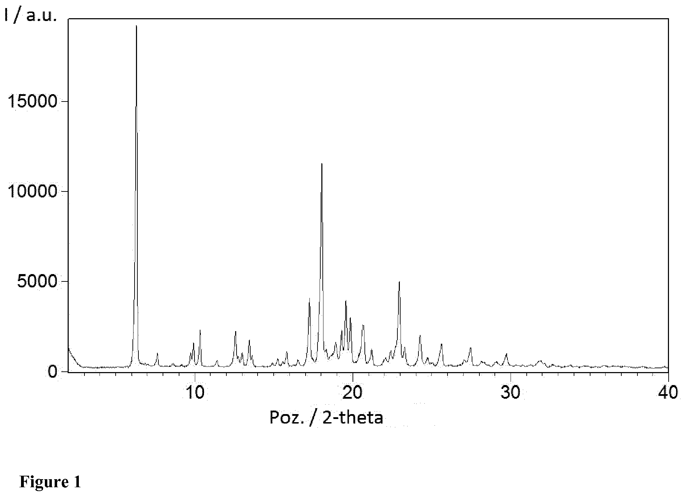

Crystalline form α of ibrutinib free base is characterized by the reflections presented in **Table 1.** Table 1 includes reflections whose relative intensity value is higher than 1%. The characteristic diffraction peaks of crystalline form α of ibrutinib free base with the use of CuKα radiation are: 6.3; 12.5; 18.0 and 22.9 ± 0.2° 2-theta. Crystalline form α further exhibits the following characteristic reflections: 10.3; 17.2; 19.5 and 24.2 ± 0.2° 2-theta. The X-ray powder pattern is shown in **Fig. 1****.**

**Table 1**

| Position [°2Th.] | Interplanar spacing [Å] [Å]=0.1nm | Rel. intensity [%] |
|---|---|---|
| 6.29 | 14.037 | 100.0 |
| 7.63 | 11.585 | 3.6 |
| 9.91 | 8.915 | 6.8 |
| 10.32 | 8.563 | 10.7 |
| 11.39 | 7.764 | 1.9 |
| 12.56 | 7.040 | 10.2 |
| 12.99 | 6.812 | 3.8 |
| 13.44 | 6.581 | 8.2 |
| 15.23 | 5.812 | 2.2 |
| 15.81 | 5.602 | 4.5 |
| 16.52 | 5.360 | 1.9 |
| 17.24 | 5.140 | 19.4 |
| 18.00 | 4.923 | 58.4 |
| 18.88 | 4.697 | 5.9 |
| 19.28 | 4.600 | 9.8 |
| 19.55 | 4.536 | 20.2 |
| 19.84 | 4.472 | 14.7 |
| 20.61 | 4.307 | 11.5 |
| 21.17 | 4.194 | 4.8 |
| 22.38 | 3.969 | 4.1 |
| 22.93 | 3.875 | 24.5 |
| 23.27 | 3.819 | 5.4 |
| 24.24 | 3.669 | 10.1 |
| 24.70 | 3.601 | 2.4 |
| 25.57 | 3.481 | 6.5 |
| 27.41 | 3.251 | 5.8 |
| 29.68 | 3.007 | 3.6 |

Differential scanning calorimetry (DSC) was applied to measure the melting point of crystalline form α of ibrutinib free base of 112°C.

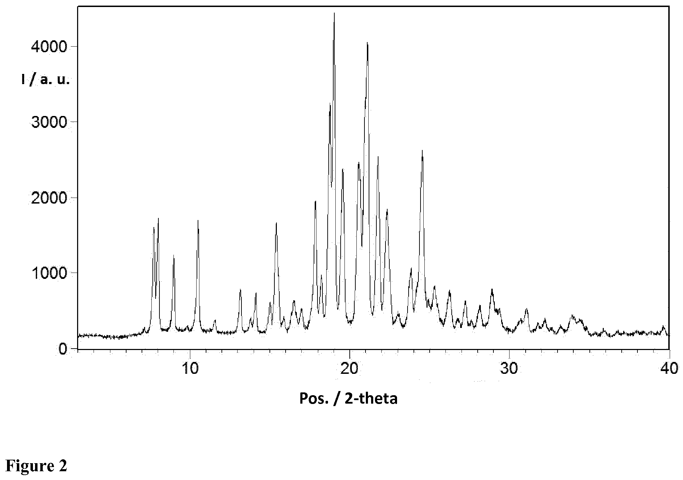

Crystalline form β of ibrutinib free base is characterized by the reflections presented in **Table 2.** Table 2 includes reflections whose relative intensity value is higher than 1%. The characteristic diffraction peaks of crystalline form β of ibrutinib free base with the use of CuKα radiation are: 8.0; 10.5; 15.4; 19.0 and 21.1 ± 0.2° 2-theta. Crystalline form β further exhibits the following characteristic reflections: 8.9; 17.8; 19.5; 21.7 and 24.5 ± 0.2° 2-theta. The X-ray powder pattern is shown in **Fig. 2****.**

**Table 2**

| Position [°2Th.] | Interplanar spacing [Å] [Å]=0.1nm | Rel. intensity [%] |
|---|---|---|
| 7.74 | 11.414 | 33.1 |
| 8.00 | 11.044 | 34.4 |
| 8.97 | 9.851 | 24.7 |
| 10.48 | 8.431 | 35.7 |
| 13.15 | 6.730 | 14.2 |
| 14.11 | 6.272 | 12.5 |
| 15.01 | 5.899 | 9.0 |
| 15.40 | 5.750 | 33.4 |
| 16.51 | 5.367 | 9.5 |
| 16.98 | 5.219 | 5.7 |
| 17.83 | 4.971 | 41.1 |
| 18.21 | 4.867 | 14.5 |
| 18.74 | 4.732 | 67.6 |
| 19.01 | 4.666 | 100.0 |
| 19.54 | 4.541 | 50.8 |
| 20.52 | 4.325 | 47.4 |
| 21.12 | 4.204 | 73.1 |
| 21.74 | 4.085 | 52.4 |
| 22.31 | 3.982 | 35.7 |
| 23.76 | 3.743 | 14.5 |
| 24.48 | 3.633 | 56.7 |
| 25.31 | 3.516 | 10.7 |
| 26.13 | 3.408 | 8.6 |
| 27.22 | 3.274 | 9.6 |
| 28.11 | 3.172 | 7.0 |
| 28.88 | 3.089 | 12.1 |
| 31.03 | 2.879 | 7.3 |

Differential scanning calorimetry (DSC) was applied to measure the melting point of crystalline form β of ibrutinib free base of 88°C.

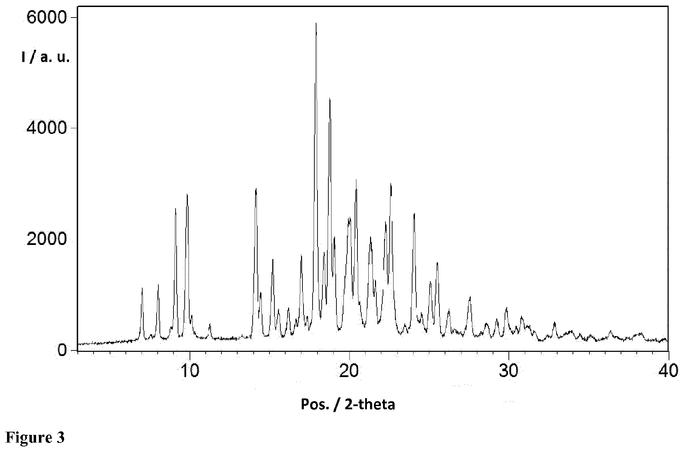

Crystalline form γ of ibrutinib free base is characterized by the reflections presented in **Table 3.** Table 3 includes reflections whose relative intensity value is higher than 1%. The characteristic diffraction peaks of crystalline form γ of ibrutinib free base with the use of CuKα radiation are: 9.8; 14.1; 17.9; 20.4 and 24.0 ± 0.2° 2-theta. Crystalline form γ further exhibits the following characteristic reflections: 9.1; 18.8 and 22.6 ± 0,2° 2-theta. The X-ray powder pattern is shown in **Fig. 3****.**

**Table 3**

| Position [°2Th.] | Interplanar spacing [Å] [Å]=0.1nm | Rel. intensity [%] |
|---|---|---|
| 7.02 | 12.588 | 17.0 |
| 8.02 | 11.020 | 18.3 |
| 9.12 | 9.694 | 40.6 |
| 9.82 | 8.996 | 48.0 |
| 11.26 | 7.854 | 4.8 |
| 14.13 | 6.264 | 48.4 |
| 14.44 | 6.128 | 14.3 |
| 15.18 | 5.834 | 24.4 |
| 15.55 | 5.694 | 8.6 |
| 16.17 | 5.478 | 9.2 |
| 16.98 | 5.218 | 25.7 |
| 17.88 | 4.956 | 100.0 |
| 18.39 | 4.821 | 25.1 |
| 18.76 | 4.727 | 76.4 |
| 19.05 | 4.654 | 29.9 |
| 19.91 | 4.455 | 32.1 |
| 20.40 | 4.351 | 49.1 |
| 21.31 | 4.166 | 31.5 |
| 21.63 | 4.106 | 14.1 |
| 22.23 | 3.995 | 33.1 |
| 22.59 | 3.932 | 48.1 |
| 24.02 | 3.702 | 42.6 |
| 25.05 | 3.552 | 18.4 |
| 25.48 | 3.493 | 24.8 |
| 26.19 | 3.400 | 8.9 |
| 27.54 | 3.237 | 9.5 |
| 28.56 | 3.122 | 4.8 |
| 29.21 | 3.055 | 6.3 |
| 29.81 | 2.995 | 10.3 |
| 30.78 | 2.902 | 6.7 |
| 32.81 | 2.727 | 5.8 |

Differential scanning calorimetry (DSC) was applied to measure the melting point of crystalline form γ of ibrutinib free base of 83°C.

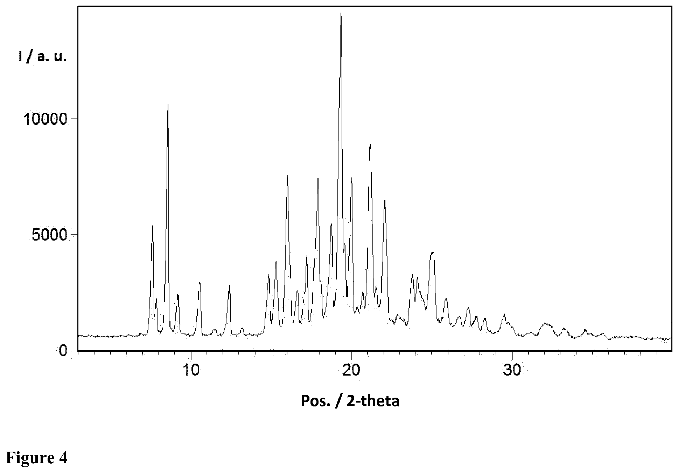

Crystalline form δ of ibrutinib free base is characterized by the reflections presented in **Table 4.** Table 4 includes reflections whose relative intensity value is higher than 1%. The characteristic diffraction peaks of crystalline form δ of ibrutinib free base with the use of CuKα radiation are: 8.5; 16.0; 19.3 and 21.1 ± 0.2° 2-theta. Crystalline form 3 further exhibits the following characteristic reflections: 7.6; 17.9; 20.0 and 22.0 ± 0.2° 2-theta. The X-ray powder pattern is shown in **Fig. 4****.**

**Table 4**

| Position [°2Th.] | Interplanar spacing [Å] [Å]=0.1nm | Rel. intensity [%] |
|---|---|---|
| 7.59 | 11.638 | 35.0 |
| 7.86 | 11.243 | 10.8 |
| 8.54 | 10.349 | 72.1 |
| 9.16 | 9.644 | 14.0 |
| 10.51 | 8.411 | 18.8 |
| 12.35 | 7.159 | 17.5 |
| 14.81 | 5.977 | 18.1 |
| 15.29 | 5.792 | 22.7 |
| 16.00 | 5.535 | 51.4 |
| 16.60 | 5.336 | 8.2 |
| 17.20 | 5.151 | 24.4 |
| 17.92 | 4.946 | 42.1 |
| 18.72 | 4.736 | 35.5 |
| 19.33 | 4.588 | 100.0 |
| 19.95 | 4.446 | 51.2 |
| 21.13 | 4.202 | 67.7 |
| 22.04 | 4.030 | 44.5 |
| 23.75 | 3.744 | 14.7 |
| 24.16 | 3.681 | 14.4 |
| 25.10 | 3.545 | 15.9 |
| 25.86 | 3.442 | 10.2 |
| 26.68 | 3.339 | 5.0 |
| 27.24 | 3.271 | 8.7 |
| 27.74 | 3.214 | 5.2 |
| 28.28 | 3.153 | 4.8 |
| 29.46 | 3.029 | 6.3 |

Differential scanning calorimetry (DSC) was applied to measure the melting point of crystalline form δ of ibrutinib free base of 90°C.

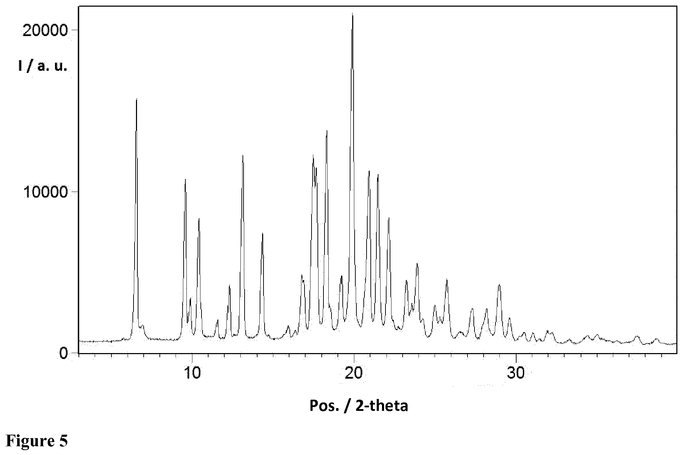

Crystalline form ε of ibrutinib free base is characterized by the reflections presented in **Table 5.** Table 5 includes reflections whose relative intensity value is higher than 1%. The characteristic diffraction peaks of crystalline form ε of ibrutinib free base with the use of CuKα radiation are: 6.5; 13.1; 17.5 and 19.9 ± 0.2° 2-theta. Crystalline form ε further exhibits the following characteristic reflections: 9.5; 18.3; 20.9 and 22.1 ± 0.2° 2-theta. The X-ray powder pattern is shown in **Fig. 5****.**

**Table 5**

| Position [°2Th.] | Interplanar spacing [Å] [Å]=0.1nm | Rel. intensity [%] |
|---|---|---|
| 6.53 | 13.534 | 74.9 |
| 9.55 | 9.254 | 50.8 |
| 9.88 | 8.950 | 12.2 |
| 10.40 | 8.498 | 37.4 |
| 11.53 | 7.666 | 5.8 |
| 12.27 | 7.208 | 17.8 |
| 13.08 | 6.763 | 56.3 |
| 14.30 | 6.172 | 32.9 |
| 16.77 | 5.282 | 20.3 |
| 16.92 | 5.237 | 15.6 |
| 17.47 | 5.073 | 58.0 |
| 17.68 | 5.014 | 48.9 |
| 18.33 | 4.837 | 65.1 |
| 19.18 | 4.623 | 19.8 |
| 19.89 | 4.460 | 100.0 |
| 20.86 | 4.254 | 52.1 |
| 21.46 | 4.138 | 53.4 |
| 22.12 | 4.015 | 39.4 |
| 23.22 | 3.828 | 19.1 |
| 23.56 | 3.774 | 8.4 |
| 23.88 | 3.723 | 25.1 |
| 25.00 | 3.560 | 10.9 |
| 25.31 | 3.516 | 7.3 |
| 25.71 | 3.462 | 19.1 |
| 27.31 | 3.263 | 8.5 |
| 28.12 | 3.170 | 9.2 |
| 28.94 | 3.083 | 19.4 |
| 29.56 | 3.017 | 7.8 |

Differential scanning calorimetry (DSC) was applied to measure the melting point of crystalline form ε of ibrutinib free base of 95°C.

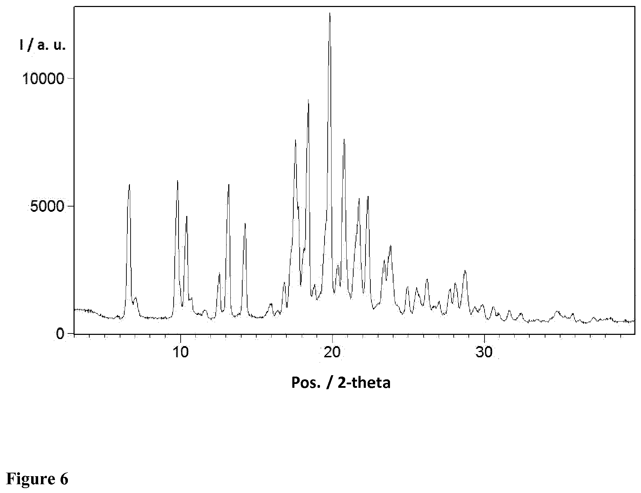

Crystalline form ζ of ibrutinib free base is characterized by the reflections presented in **Table 6.** Table 6 includes reflections whose relative intensity value is higher than 1%. The characteristic diffraction peaks of crystalline form ζ of ibrutinib free base with the use of CuKα radiation are: 6.6; 13.1; 17.6 and 19.8 ± 0.2° 2-theta. Crystalline form ζ further exhibits the following characteristic reflections: 9.8; 18.4; 20.7; 22.3 and 23.8 ± 0.2° 2-theta. The X-ray powder pattern is shown in **Fig. 6****.**

**Table 6**

| Position [°2Th.] | Interplanar spacing [Å] [Å]=0.1nm | Rel. intensity [%] |
|---|---|---|
| 6.58 | 13.418 | 44.9 |
| 9.76 | 9.054 | 42.2 |
| 10.37 | 8.521 | 33.7 |
| 12,52 | 7.063 | 13.5 |
| 13.12 | 6.740 | 42.3 |
| 14.22 | 6.222 | 30.5 |
| 16.80 | 5.272 | 9.9 |
| 17.57 | 5.044 | 54.9 |
| 18.39 | 4.820 | 71.8 |
| 19.79 | 4.483 | 100.0 |
| 20.32 | 4.367 | 14.2 |
| 20.74 | 4.279 | 56.1 |
| 21.72 | 4.088 | 31.3 |
| 22.31 | 3.982 | 38.7 |
| 23.35 | 3.807 | 13.7 |
| 23.78 | 3.738 | 21.7 |
| 24.93 | 3.569 | 9.6 |
| 25.58 | 3.479 | 8.5 |
| 26.21 | 3.398 | 12.3 |
| 27.70 | 3.218 | 9.0 |
| 28.10 | 3.173 | 11.0 |
| 28.72 | 3.106 | 16.5 |
| 29.84 | 2.992 | 4.9 |
| 30.58 | 2.921 | 4.8 |
| 31.65 | 2.825 | 3.6 |

Differential scanning calorimetry (DSC) was applied to measure the melting point of crystalline form ζ of ibrutinib free base of 99°C.

The solid forms of ibrutinib free base prepared according to this invention can be used for the preparation of pharmaceutical compositions, especially solid drug forms, e.g. tablets or capsules. Such pharmaceutical compositions can contain at least one excipient from the group of fillers (e.g. lactose), binders (e.g. microcrystalline cellulose), disintegrants (e.g. sodium salt of croscarmellose), lubricants (e.g. magnesium stearate), surfactants, etc. Ibrutinib free base can be mixed with the above mentioned excipients, screened through a sieve and the resulting mixture can be tabletted or filled into capsules. The tablets can be further coated with common coating compounds, e.g. polyvinyl alcohol or polyethylene glycol.

The term "room temperature" refers, for the purposes of the text below and above, to the temperature range from 22 to 26°C.

The invention is clarified in a more detailed way using the embodiment examples below. These examples, which illustrate the preparation of solid forms of ibrutinib free base, only have an illustrative character and do not restrict the scope of the invention in any respect.

### Experimental part

### X-ray powder diffraction

The diffraction patterns were obtained using an X'PERT PRO MPD PANalytical powder diffractometer, used radiation CuKα (λ=0.1542 nm (1.542 Å)), excitation voltage: 45 kV, anode current: 40 mA, measured range: 2 - 40° 2θ, increment: 0.01° 2θ at the dwell time at a reflection of 0.5 s, the measurement was carried out with a flat sample with the area/thickness of 10/0.5 mm. For the correction of the primary array 0.02 rad Soller slits, a 10mm mask and a 1/4° fixed anti-dispersion slit were used. The irradiated area of the sample is 10 mm, programmable divergence slits were used. For the correction of the secondary array 0.02 rad Soller slits and a 5.0 mm anti-dispersion slit were used.

### Differential Scanning Calorimetry (DSC)

The records of the solid crystalline forms of ibrutinib were measured with the use of a DSC Pyris 1 device by Perkin Elmer. The sample charge in a standard A1 pot was between 2.5 and 3 mg and the heating rate was 10°C/min. The temperature program that was used consists of 1 min of stabilization at the temperature of 0°C and then of heating up to 300°C at the heating rate of 10°C/min. As the carrier gas 4.0 N₂ was used at the flow of 20 ml/min.

### Measurement using the true dissolution method

Dissolution from disks of ibrutinib (i.e. true dissolution) was carried out using a 708-DS device and measured with a Cary 60 spectrophotometer, both made by Agilent Technologies. The sample charge in the disk mold was between 40 and 45 mg. A disk with an exactly defined surface area of 0.125 cm² was then produced by compression of the sample with the force of 2 tons, maintaining this load for 30 seconds and subsequent release. This step was subsequently repeated once again. Dissolution from the disk for individual samples was carried out in 10 mM HCl (pH = 2.0) with the volume of 500 ml and temperature of 37°C. The concentration of dissolved ibrutinib was measured in 5mm cuvettes at the wavelength of 288 nm. The rotational speed of the disk was 100 rpm.

### Examples

The crystalline form of ibrutinib free base (form A) was prepared according to the process disclosed in patent application WO2013/184572.

The crystalline form of ibrutinib free base (form C) was prepared according to the process disclosed in patent application WO2013/184572.

### Example 1

### Preparation of crystalline form α of ibrutinib free base in dioxolane

The crystalline free base of 1-[(3*R*)-3-[4-amino-3-(4-phenoxyphenyl)-1H-pyrazolo[3,4-d]pyrimidin-1-yl]piperidin-1-yl]prop-2-en-1-one (form A) in the amount of 50 mg was dissolved in 0.1 ml of dioxolane at 70°C. The resulting solution was abruptly cooled down to -20°C. The obtained solid phase was filtered and characterized by means of X-ray powder diffraction (Fig. 1). Melting point according to DSC: 112°C.

### Example 2

### Preparation of crystalline form α of ibrutinib free base in dioxolane

The crystalline free base of 1-[(3*R*)-3-[4-amino-3-(4-phenoxyphenyl)-1H-pyrazolo[3,4-d]pyrimidin-1-yl]piperidin-1-yl]prop-2-en-1-one (form C) in the amount of 500 mg was suspended in 1 ml of the saturated solution of ibrutinib in dioxolane. The obtained suspension was left to be shaken at the room temperature for 48 h and subsequently the solid fraction was filtered. The obtained product was left to dry at the room temperature and pressure for 12 h. The X-ray powder pattern is shown in Fig. 2. Melting point according to DSC: 112°C.

### Example 3

### Preparation of crystalline form β of ibrutinib free base in α,α,α-trifluorotoluene

The crystalline free base of 1-[(3*R*)-3-[4-amino-3-(4-phenoxyphenyl)-1H-pyrazolo[3,4-d]pyrimidin-1-yl]piperidin-1-yl]prop-2-en-1-one (form A) in the amount of 50 mg was dissolved in 1 ml of *α,α,α*-trifluorotoluene at 100°C. The resulting solution was abruptly cooled down to -20°C. The separated solid fraction was stored at this temperature for 3 days. The separated solid fraction was filtered. The obtained product was left to dry at a reduced pressure of 200 mbar at the temperature of 40°C for 12 h. The X-ray powder pattern is shown in Fig. 2. Melting point according to DSC: 88°C.

### Example 4

### Preparation of crystalline form β of ibrutinib free base in α,α,α-trifluorotoluene

The crystalline free base of 1-[(3*R*)-3-[4-amino-3-(4-phenoxyphenyl)-1H-pyrazolo[3,4-d]pyrimidin-1-yl]piperidin-1-yl]prop-2-en-1-one (form C) in the amount of 500 mg was suspended in 1 ml of the saturated solution of ibrutinib in *α,α,α*-trifluorotoluene. The obtained suspension was left to be shaken at the room temperature for 48 h and subsequently the solid fraction was filtered. The obtained product was left to dry at a reduced pressure of 200 mbar at the temperature of 40°C for 12 h. The X-ray powder pattern is shown in Fig. 2. Melting point according to DSC: 88°C.

### Example 5

### Preparation of crystalline form γ of ibrutinib free base in 4-hydroxy-4-methylpentan-2-one

The crystalline free base of 1-[(3*R*)-3-[4-amino-3-(4-phenoxyphenyl)-1H-pyrazolo[3,4-d]pyrimidin-1-yl]piperidin-1-yl]prop-2-en-1-one (form A) in the amount of 50 mg was dissolved in 50 µl of 4-hydroxy-4-methylpentan-2-one at 120°C. The resulting solution was abruptly cooled down to -20 °C. The separated solid fraction was stored at this temperature for 3 days. The separated solid fraction was filtered. The obtained product was left to dry at a reduced pressure of 200 mbar at the temperature of 40°C for 12 h. The X-ray powder pattern is shown in Fig. 3. Melting point according to DSC: 83°C.

### Example 6

### Preparation of crystalline form γ of ibrutinib free base in 4-hydroxy-4-methylpentan-2-one

The crystalline free base of 1-[(3*R*)-3-[4-amino-3-(4-phenoxyphenyl)-1H-pyrazolo[3,4-d]pyrimidin-1-yl]piperidin-1-yl]prop-2-en-1-one (form C) in the amount of 500 mg was suspended in 1 ml of the saturated solution of ibrutinib in 4-hydroxy-4-methylpentan-2-one. The obtained suspension was left to be shaken at the room temperature for 48 h and subsequently the solid fraction was filtered. The obtained product was left to dry at a reduced pressure of 200 mbar at the temperature of 40°C for 12 h. The X-ray powder pattern is shown in Fig. 3. Melting point according to DSC: 83°C.

### Example 7

### Preparation of crystalline form δ of ibrutinib free base in ortho-xylene

The crystalline free base of 1-[(3*R*)-3-[4-amino-3-(4-phenoxyphenyl)-1H-pyrazolo[3,4-d]pyrimidin-1-yl]piperidin-1-yl]prop-2-en-1-one (form C) in the amount of 500 mg was suspended in 1 ml of the saturated solution of ibrutinib in 4-hydroxy-4-methylpentan-2-one. The obtained suspension was left to be shaken at the room temperature for 48 h and subsequently the solid fraction was filtered. The obtained product was left to dry at a reduced pressure of 200 mbar at the temperature of 40°C for 12 h. The X-ray powder pattern is shown in Fig. 4. Melting point according to DSC: 90°C.

### Example 8

### Preparation of crystalline form ε of ibrutinib free base in meta-xylene

The crystalline free base of 1-[(3*R*)-3-[4-amino-3-(4-phenoxyphenyl)-1H-pyrazolo[3,4-d]pyrimidin-1-yl]piperidin-1-yl]prop-2-en-1-one (form C) in the amount of 500 mg was suspended in 1 ml of the saturated solution of ibrutinib in *meta*-xylene. The obtained suspension was left to be shaken at the room temperature for 48 h and subsequently the solid fraction was filtered. The obtained product was left to dry at a reduced pressure of 200 mbar at the temperature of 40°C for 12 h. The X-ray powder pattern is shown in Fig. 5. Melting point according to DSC: 95°C.

### Example 9

### Preparation of crystalline form ζ of ibrutinib free base in para-xylene

The crystalline free base of 1-[(3*R*)-3-[4-amino-3-(4-phenoxyphenyl)-1H-pyrazolo[3,4-d]pyrimidin-1-yl]piperidin-1-yl]prop-2-en-1-one (form C) in the amount of 500 mg was suspended in 1 ml of the saturated solution of ibrutinib in *para*-xylene. The obtained suspension was left to be shaken at the room temperature for 48 h and subsequently the solid fraction was filtered. The obtained product was left to dry at a reduced pressure of 200 mbar at the temperature of 40°C for 12 h. The X-ray powder pattern is shown in Fig. 6. Melting point according to DSC: 99°C.

## Claims

1. A solid crystalline form of the ibrutinib free base, selected from the group consisting of forms γ, β, α, δ, ε and ζ, obtainable by recrystallization of the crystalline form A, **characterized by** the X-ray powder pattern with the use of CuKα radiation having the following reflections: 5.7, 13.6, 16.1 18.9, 21.3, and 21.6±0.1° 2-theta, or of the crystalline form C, **characterized by** the following reflections: 7.0, 14.0, 15.7, 18.2, 19.1, 19.5, 20.3, 22.1 and 22.9±0.1° 2-theta, from an aliphatic, alicyclic or aromatic solvent.

2. Crystalline form γ of ibrutinib free base according to claim 1, exhibiting the following characteristic reflections in the X-ray powder pattern with the use of CuKα radiation: 9.8; 14.1; 17.9; 20.4 and 24.0 ± 0.2° 2-theta.

3. A process for preparing crystalline form γ of ibrutinib free base according to any one of claims 1 or 2, **characterized in that** the process comprises:
a) dissolving ibrutinib free base in 4-hydroxy-4-methylpentan-2-one at a temperature in the range from 30°C to the boiling point of the solvent;
b) crystallizing the solution (cooling, evaporation of the solvent), or suspending the solid free base of ibrutinib in the prepared solution;
c) isolating the solid fraction by filtration and its drying.

4. Crystalline form β of ibrutinib free base according to claim 1, exhibiting the following characteristic reflections in the X-ray powder pattern with the use of CuKα radiation: 8.0; 10.5; 15.4; 19.0 and 21.1 ± 0.2° 2-theta.

5. A process for preparing crystalline form β of ibrutinib free base according to any one of claims 1 or 4, **characterized in that** the process comprises:
a) dissolving ibrutinib free base in *α,α,α*-trifluorotoluene at a temperature in the range from 30°C to the boiling point of the solvent;
b) crystallizing the solution (cooling, evaporation of the solvent), or suspending the solid free base of ibrutinib in the prepared solution;
c) isolating the solid fraction by filtration and its drying.

6. Crystalline form α of ibrutinib free base according to claim 1, exhibiting the following characteristic reflections in the X-ray powder pattern with the use of CuKα radiation: 6.3; 12.5; 18.0 and 22.9 ± 0.2° 2-theta.

7. A process for preparing crystalline form α of ibrutinib free base according to any one of claims 1 or 6, **characterized in that** the process comprises:
a) dissolving ibrutinib free base in dioxolane at a temperature in the range from 30°C to the boiling point of the solvent;
b) crystallizing the solution (cooling, evaporation of the solvent), or suspending the solid free base of ibrutinib in the prepared solution;
c) isolating the solid fraction by filtration and its drying.

8. Crystalline form δ of ibrutinib free base according to claim 1, exhibiting the following characteristic reflections in the X-ray powder pattern with the use of CuKα radiation: 8.5; 16.0; 19.3 and 21.1 ± 0.2° 2-theta.

9. A process for preparing crystalline form δ of ibrutinib free base according to any one of claims 1 or 8, **characterized in that** the process comprises:
a) dissolving ibrutinib free base in *ortho*-xylene at a temperature in the range from 30°C to the boiling point of the solvent;
b) crystallizing the solution (cooling, evaporation of the solvent), or suspending the solid free base of ibrutinib in the prepared solution;
c) isolating the solid fraction by filtration and its drying.

10. Crystalline form ε of ibrutinib free base according to claim 1, exhibiting the following characteristic reflections in the X-ray powder pattern with the use of CuKα radiation: 6.5; 13.1; 17.5 and 19.9 ± 0.2° 2-theta.

11. A process for preparing crystalline form ε of ibrutinib free according to any one of claims 1 or 10, **characterized in that** the process comprises:
a) dissolving ibrutinib free base in *meta*-xylene at a temperature in the range from 30°C to the boiling point of the solvent;
b) crystallizing the solution (cooling, evaporation of the solvent), or suspending the solid free base of ibrutinib in the prepared solution;
c) isolating the solid fraction by filtration and its drying.

12. Crystalline form ζ of ibrutinib free base according to claim 1, exhibiting the following characteristic reflections in the X-ray powder pattern with the use of CuKα radiation: 6.6; 13.1; 17.6 and 19.8 ± 0.2° 2-theta.

13. A process for preparing crystalline form ζ of ibrutinib free base according to any one of claims 1 or 12, **characterized in that** the process comprises:
a) dissolving ibrutinib free base in *para*-xylene at a temperature in the range from 30°C to the boiling point of the solvent;
b) crystallizing the solution (cooling, evaporation of the solvent), or suspending the solid free base of ibrutinib in the prepared solution;
c) isolating the solid fraction by filtration and its drying.
